(19) Europäisches Patentamt
European Patent Office
Office européen des brevets

(11) **EP 3 288 503 B1**

(12) **EUROPÄISCHE PATENTSCHRIFT**

(45) Veröffentlichungstag und Bekanntmachung des Hinweises auf die Patenterteilung:
**20.09.2023   Patentblatt 2023/38**

(21) Anmeldenummer: **16718708.7**

(22) Anmeldetag: **27.04.2016**

(51) Internationale Patentklassifikation (IPC):
**A61F 2/66** *(2006.01)*

(52) Gemeinsame Patentklassifikation (CPC):
**A61F 2/66; A61F 2/6607; A61F 2/74; A61F 2/748; A61F 2/76;** A61F 2/5044; A61F 2002/6845

(86) Internationale Anmeldenummer:
**PCT/EP2016/059415**

(87) Internationale Veröffentlichungsnummer:
**WO 2016/174096 (03.11.2016 Gazette 2016/44)**

(54) **FUSSPROTHESE**

PROSTHETIC FOOT

PROTHÈSE DE PIED

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2015   DE 102015207936**

(43) Veröffentlichungstag der Anmeldung:
**07.03.2018   Patentblatt 2018/10**

(73) Patentinhaber: **Ottobock SE & Co. KGaA**
**37115 Duderstadt (DE)**

(72) Erfinder:
• **KALTENBORG, Sven**
**37115 Duderstadt (DE)**
• **GEHRMANN, Georg**
**37085 Göttingen (DE)**
• **SCHWARTZ, Miclas**
**37083 Göttingen (DE)**

(74) Vertreter: **Gramm, Lins & Partner Patent- und Rechtsanwälte PartGmbB Frankfurter Straße 3 C 38122 Braunschweig (DE)**

(56) Entgegenhaltungen:
**EP-A1- 1 974 699     WO-A1-2014/057086**

**Beschreibung**

Die Erfindung betrifft eine Fußprothese mit einem Unterschenkel-Anschlussteil, einem Fußteil, einem Verbindungsteil mit Gelenkfunktion, das das Unterschenkel-Anschlussteil mit dem Fußteil verbindet, und einer Freigabevorrichtung, mittels der eine Bewegung des Fußteils relativ zu dem Unterschenkel-Anschlussteil hemmbar und enthemmbar ist, die eine passive Hemmvorrichtung (28) besitzt, und die einen Hydraulikzylinder, der mit dem Fußteil und dem Unterschenkel-Anschlussteil verbunden ist, aufweist.

Eine derartige Prothese ist aus der WO2014/057086 A1 bekannt, bei der eine Dämpfung eines um ein Knöchelgelenk verschwenkbaren Fußteils über einen ersten Hydraulikzylinder einstellbar ist, wobei dieser wiederum von einem zweiten Hydraulikzylinder beaufschlagbar ist. Nachteilig an dieser Prothese ist, dass zum Vermeiden eines Stolperns ein Vorderfußteil vorgesehen und angehoben werden muss, was aufwendig ist.

Aus der EP 1 974 699 A1 ist ein Prothesen- oder Orthesengelenk bekannt, bei dem eine schwenkwinkelabhängige Dämpfung über an einen Drehkolben eingearbeitete Überströmkonturen erreicht wird. Nachteilig an einem derartigen Gelenk ist der komplexe Aufbau und der hohe Fertigungsaufwand,

Aus der US 8,709,091 B2 ist eine Prothese bekannt, bei der ein Mikroprozessor anhand von Sensordaten erfasst, wann der Gangablauf so weit fortgeschritten ist, dass das Gelenk freigeschaltet werden muss. Nachteilig an einer derartigen Prothese ist der hohe Aufwand bei der Fertigung und der Wartung.

Aus der WO 2014/039885 A1 ist eine Prothese bekannt, bei der ebenfalls mittels eines Mikroprozessors und einer Vielzahl von Sensoren der Gangablauf erfasst wird. In einer Steuerung der Prothese können Schwellenwerte hinterlegt werden, anhand derer die Aktoren der Prothese angesteuert werden. Auch ein derartiges System ist aufwendig zu fertigen und zu warten.

Aus der US 2014/0074255 A1 ist ein künstlicher Fuß bekannt, bei dem ein rollfähiges Element verwendet wird, um einen Flüssigkeitskanal genau dann zu verschließen, wenn der Patient steht. Auf diese Weise wird eine Dorsalextensionsbewegung des Fußes dann gestoppt, wenn sich der Schwerpunkt oberhalb des Fußes befindet. Nachteilig an einem derartigen Fuß ist die erhöhte Stolpergefahr.

Aus der US 2014/0330393 A1 ist ein Fuß bekannt, bei dem zwei Federn dazu verwendet werden, um eine Dorsalextensionsbewegung bis zu einer Nullstellung mit einem ersten Drehmoment zu beaufschlagen und danach mit einem zweiten Drehmoment zu beaufschlagen. Nachteilig an einem derartigen System ist die Geräuschentwicklung.

Nachteilig an bekannten Prothesen ist zudem, dass es vergleichsweise aufwendig ist, die Prothese anzupassen, wenn Schuhe mit unterschiedlichen Absatzhöhen getragen werden.

Der Erfindung liegt die Aufgabe zugrunde, Nachteile im Stand der Technik zu vermindern.

Die Erfindung löst das Problem durch eine Fußprothese, deren Hemmvorrichtung so mit dem Hydraulikzylinder verbunden ist, , dass eine Dorsalextensionsbewegung des Fußteils relativ zum Unterschenkel-Anschlussteil in einem Winkelbereich von einer Maximal-Plantarflexionsstellung bis zu einer Nullstellung wenig stark gehemmt wird als eine Dorsal-Extensionsbewegung des Fußteils aus der Nullstellung, und eine Dorsalextensionsbewegung über die Nullstellung hinaus möglich ist. Vorzugsweise ist die Hemmvorrichtung so ausgebildet, dass die Nullstellung einstellbar ist. Die Maximal-Plantarflexionsstellung ist diejenige Stellung, in der das Fußteil maximal plantarflektiert ist.

Vorteilhaft an einer derartigen Fußprothese ist, dass sie besonders einfach aufgebaut ist. So sind eine Steuerung und Sensoren sowie Aktoren entbehrlich.

Gegenüber beispielsweise mechanischen Hemmvorrichtungen hat eine hydraulische Hemmvorrichtung den Vorteil, dass sie besonders leise arbeitet.

Dadurch, dass die Dorsal-Extensionsbewegung zwischen der Maximal-Plantarflexionsstellung und der Nullstellung weniger stark gehemmt wird als über die Nullstellung hinaus, führt dass das Fußteil nach dem Aufsetzen der Ferse zunächst ohne einen großen Widerstand zu leisten relativ zum Unterschenkel-Anschlussteil schwenken kann. Sobald sich der Schwerpunkt des Patienten, der die Fußprothese trägt, über der Fußprothese befindet, wird eine weitere dorsale Extensionsbewegung hingegen stärker gehemmt oder sogar vollständig unterbunden. Das führt dazu, dass der Körper des Patienten nicht an Höhe verliert.

Hat sich der Schwerpunkt des Körpers des Patienten über den Ballenbereich der Fußprothese bewegt, so überschreitet das Knöchel-Drehmoment einen Freigabe-Schwellenwert und die Hemmvorrichtung gibt gemäß einer bevorzugten Ausführungsform der Prothese die Dorsal-Extensionsbewegung des Fußteils frei. Beim nachfolgenden Abheben der Fußprothese ergibt sich dadurch ein größerer Abstand zwischen dem Boden und der Spitze der Fußprothese, sodass die Gefahr des Stolperns verringert wird. Setzt die Fußprothese zu Beginn des nachfolgenden Gangzyklus auf den Boden auf, so führt das Fußteil eine Plan tarflexionsbewegung aus und der oben beschriebene Ablauf wird erneut durchlaufen.

Im Rahmen der vorliegenden Beschreibung wird unter dem Unterschenkel-Anschlussteil insbesondere eine Vorrichtung verstanden, die ausgebildet ist zum direkten oder indirekten mechanischen Verbinden mit einem künstlichen oder natürlichen Unterschenkel. Es ist also möglich, nicht aber notwendig, dass ein künstlicher Unterschenkel und/oder ein künstliches Knöchelgelenk vorhanden ist, der unlösbar oder lösbar mit dem Unterschenkel-Anschlussteil verbunden ist.

Unter dem Fußteil wird insbesondere derjenige Teil der Prothese verstanden, der die Funktion des menschlichen Fußes übernimmt. Insbesondere kann der Fußteil eine Fußkosmetik umfassen, die der Prothese eine Anmutung eines natürlichen Fußes gibt. Eine derartige Fußkosmetik ist jedoch entbehrlich.

Unter dem Verbindungselement wird insbesondere eine Verbindung zwischen dem Unterschenkel-Anschlussteil und dem Fußteil verstanden, die eine Relativbewegung der beiden Komponenten erlaubt, die der Bewegung eines natürlichen Fußes entspricht. Das Verbindungselement kann ein Drehgelenk aufweisen oder durch ein Drehgelenk gebildet sein, das eine feste Drehachse aufweist. Diese Lage der Drehachse kann vom Drehwinkel des Drehgelenks unabhängig sein.

Es ist aber auch möglich, dass das Verbindungselement mehrere Teil-Gelenke aufweist und eine momentane Drehachse hat, das heißt, dass sich die Drehachse, mittels der die momentane Drehung des Fußteils relativ zum Unterschenkel-Anschlussteil beschreibbar ist, sich mit der Zeit und/oder dem Fortschritt der Bewegung im Gangzyklus ändert. Es ist auch möglich, dass das Verbindungselement zumindest teilweise als Festkörpergelenk aufgebaut ist. Die meisten Prothesen zielen darauf ab, den natürlichen Bewegungsablauf möglichst gut zu imitieren. Da der menschliche Fuß mehrere Teil-Gelenke hat, besitzen viele Prothesen ein Verbindungselement, das eine mit der Zeit in seiner Lage verändernde Drehachse besitzt. Die Erfindung bezieht sich auch auf Prothesen mit solchen Verbindungselementen.

Unter einer Dorsal-Extensionsbewegung des Fußteils aus der Nullstellung wird eine Schwenkbewegung des Fußteils verstanden, die in Dorsal-Extensionsrichtung über die Nullstellung hinaus erfolgt.

Vorzugsweise verbindet das Verbindungselement das Unterschenkel-Anschlussteil wie ein Knöchelgelenk mit dem Fußteil. Hierunter ist insbesondere zu verstehen, dass das Verbindungselement die Bewegung des menschlichen Knöchelgelenks nachbildet. Es kann sich daher bei dem Verbindungselement um ein Drehgelenk handeln, das ist aber nicht notwendig.

Vorzugsweise ist das Verbindungselement so ausgebildet, dass es einer Dorsal- und/oder Plantar-Bewegung einen, beispielsweise um zumindest den Faktor fünf, geringeren Widerstand entgegensetzt als einer Medial- oder Lateralbewegung.

Vorzugsweise ist das Fußteil relativ zum Unterschenkel-Anschlussteil schwenkbar. Darunter wird insbesondere verstanden, dass die Freigabevorrichtung eine Bewegung zwischen Fußteil und Unterschenkel-Anschlussteil so wenig behindert, dass das Fußteil im normalen Gangzyklus eine Dorsalextensionsbewegung ausführt oder ausführen kann. Vorzugsweise ist die Freigabevorrichtung so ausgebildet, dass sie einer Dorsalextensionsbewegung des Fußteils relativ zum Unterschenkel-Anschlussteil über die Nutstellung hinaus einen zumindest dreifach so hohen Widerstand entgegensetzt wie zwischen der Maximal-Plantarflexionsstellung.

Unter dem Merkmal, dass die Hemmvorrichtung eine passive Hemmvorrichtung ist, wird insbesondere verstanden, dass das Umschalten ohne einen Aktor erfolgt. Ein Aktor ist ein Bauteil, das von außen steuerbar durch Energieeinsatz ein anderes Bauteil bewegt. Insbesondere ist die Hemmvorrichtung aktorfrei und/oder energiespeicherlos. Vorzugsweise ist die Hemmvorrichtung so aufgebaut, dass die Energie, die zum Übergang von einer Freigabestellung, in der die Dorsalextensionsbewegung weniger stark gehemmt ist, in eine Freigabestellung, in der die Dorsalextensionsbewegung stärker gehemmt ist, notwendig ist, mechanische, insbesondere hydraulische Energie ist. Insbesondere ist die Freigabevorrichtung eine passive hydraulische Freigabevorrichtung.

Unter der Nullstellung wird insbesondere diejenige Stellung des Fußteils relativ zum Unterschenkel-Anschlussteil verstanden, bei der der Übergang stattfindet von einer weniger stark gehemmten Schwenkbarkeit in Dorsalextensionsrichtung zu einer stärker gehemmten, insbesondere unterdrückten, Schwenkbarkeit in Dorsalextensionsrichtung. Es sei darauf hingewiesen, dass es möglich, nicht aber notwendig ist, dass die Maximal-Plantarflexionsstellung im Gangablauf erreicht wird. Vorzugsweise ist die Nullstellung aber so gewählt, dass im Gangablauf eine Stellung des Fußteils zwischen der Nullstellung und der Maximal-Plantarflexionsstellung erreicht wird und/oder dass das Fußteil in der Nullstellung ist, wenn der Patient steht.

In anderen Worten lassen sich zwei Winkelbereiche unterscheiden: die Winkel zwischen der Maximal-Plantarflexionsstellung und der Nullstellung und die Winkel von der Nullstellung bis zu einer Maximal-Dorsalextensionsstellung. Im erstgenannten Winkelbereich ist eine Dorsalextensionsbewegung leicht möglich, im zweitgenannten Winkelbereich deutlich schwerer oder nicht.

Unter dem Merkmal, dass der Hydraulikzylinder mit dem Fußteil und dem Unterschenkel-Anschlussteil verbunden ist, wird insbesondere verstanden, dass ein bewegliches Teil des Hydraulikzylinders, also das Gehäuse oder die Kolbenstange, mit dem Fußteil und das jeweils andere bewegliche Teil mit dem Unterschenkel-Anschlussteil verbunden ist.

Gemäß einer bevorzugten Ausführungsform ist die Freigabevorrichtung so ausgebildet, dass sie die Dorsalextensionsbewegung des Fußteils über die Nullstellung und nur dann freigibt, wenn ein Knöchel-Drehmoment, das am Fußteil anliegt, oberhalb eines vorgegebenen Freigabe-Schwellenwerts liegt. Das hat den Vorteil, dass es, wie oben beschrieben, am Ende der Standphase im Gangzyklus zu einer Dorsalextensionsbewegung des Fußteils relativ zum Unterschenkel-Anschlussteil über die Nullstellung hinaus kommt, was in der nachfolgenden Schwungphase einen Abstand zwischen der Spitze des Fußes und dem Boden verringert und so die Stolpergefahr minimiert.

Es ist möglich, aber entbehrlich, dass der Drehmoment-Schwellenwert als Drehmoment bekannt ist. Insbesondere ist es möglich, dass der Drehmoment-Schwellenwert hin zu größeren und/oder kleineren Drehmoment-Schwellenwerten veränderbar ist, ohne dass der absolute Wert des Drehmoments bekannt ist. Wie groß der jeweilige Drehmoment-Schwellenwert in Newtonmeter ist, kann also bekannt sein, das ist aber nicht notwendig.

Der Drehmoment-Schwellenwert kann insbesondere ermittelt werden, indem eine lotrecht wirkende Kraft auf das Unterschenkel-Anschlussteil ausgeübt, danach das Unterschenkel-Anschlussteil gemäß dem Neigungsverlauf im Gangzyklus geneigt und der Punkt erfasst wird, in dem die Prothese die Kraft in den Boden einleitet. Die Projektion des (äußeren) Vektorprodukts aus der Kraft und dem Hebelarm zu dem Zeitpunkt, in dem die Freigabevorrichtung aus der Arretierstellung in die Freigabestellung auf die Drehachse schaltet, entspricht dem Drehmoment-Schwellenwert, der positiv ist.

Die Freigabevorrichtung kann parallel oder seriell zu einem weiteren federnden Element der Prothese angeordnet sein. Beispielsweise ist das federnde Element Teil des Fußteils. Das federnde Element umfasst vorzugsweise eine Karbonfeder. Unter einer parallelen Anordnung wird verstanden, dass sich die mechanischen Widerstände der Freigabevorrichtung und des federnden Elements addieren. Unter einer seriellen Anordnung wird verstanden, dass sich die Federwege der Freigabevorrichtung und des federnden Elements addieren.

Die Freigabevorrichtung kann druckbelastet oder zugbelastet angeordnet sein. Ist die Freigabevorrichtung druckbelastet, so wird sie am Ende der Standphase zusammengedrückt. Ist die Freigabevorrichtung zugbelastet, so wird sie am Ende der Standphase auseinandergezogen.

Gemäß einer bevorzugten Ausführungsform umfasst die Prothese eine Vorspannfeder, die so angeordnet ist, dass sie beim Fersenauftritt im Anschlag ist.

Gemäß einer bevorzugten Ausführungsform ist die Hemmvorrichtung eine hydraulische Hemmvorrichtung. Das heißt insbesondere, dass die hemmende Wirkung der Hemmvorrichtung zumindest auch, insbesondere ausschließlich, durch hydraulische Komponenten bewirkt wird.

Erfindungsgemäß weist die Freigabevorrichtung einen Hydraulikzylinder, der mit dem Fußteil und dem Unterschenkel-Anschlussteil verbunden ist, auf und die Hemmvorrichtung ist so mit dem Hydraulikzylinder verbunden, dass eine Dorsalextensionsbewegung des Fußteils relativ zum Unterschenkel-Anschlussteil in einem Winkelbereich von einer Maximal-Plantarflexionsstellung, in der das Fußteil in der maximal plantarflektierten Stellung ist, bis zu einer Nullstellung weniger stark gehemmt wird als eine Dorsalextensionsbewegung des Fußteils aus der Nullstellung. Eine derartige Fußprothese ist leise und wartungsarm.

Erfindungsgemäß ist insbesondere eine Fußprothese mit (a) einem Unterschenkel-Anschlussteil,(b) einem Fußteil, (c) einem Verbindungselement mit Gelenkfunktion, das das Unterschenkel-Anschlussteil mit dem Fußteil verbindet, und (d) einer Freigabevorrichtung, mittels der eine Bewegung des Fußteils relativ zum Unterschenkel-Anschlussteil hemmbar und enthemmbar ist, wobei (e) die Freigabevorrichtung eine passive hydraulische Hemmvorrichtung besitzt, die so ausgebildet ist, dass eine Dorsalextensionsbewegung des Fußteils relativ zum Unterschenkel-Anschlussteil in einem Winkelbereich von einer Maximal-Plantarflexionsstellung, in der das Fußteil in der maximal plantarflektierten Stellung ist, bis zu einer Nullstellung weniger stark gehemmt wird als eine Dorsalextensionsbewegung des Fußteils aus der Nullstellung, wobei diese Fußprothese vorzugsweise die im vorigen Absatz beschriebene Freigabevorrichtung aufweist. Die in der vorliegenden Beschreibung dargelegten bevorzugten Ausführungsformen beziehen sich auch auf diese Erfindung.

Vorzugsweise besitzt die Hemmvorrichtung eine erste Fluidleitung, durch die Hydraulikfluid fließt, wenn das Fußteil eine Dorsalextionsbewegung über die Nullstellung hinaus ausführt, wobei die erste Fluidleitung einen ersten Strömungswiderstand hat, wobei die Hemmvorrichtung zudem eine Überbrückungsleitung besitzt, über die das Hydraulikfluid fließt, wenn das Fußteil eine Dorsalextionsbewegung zwischen der Maximal-Plantarflexionsstellung und der Nullstellung ausführt, wobei die Überbrückungsleitung einen Überbrückungs-Strömungswiderstand hat, der kleiner ist als der ersten Strömungswiderstand. Hierunter wird insbesondere bestanden, dass der Überbrückungs-Strömungswiderstand höchstens ein Drittel, insbesondere höchstens ein Fünftel, des ersten Strömungswiderstands beträgt. Auf diese Weise ist eine Plantarflexionsbewegung zwischen der Maximal-Plantarflexionsstellung und der Nullstellung deutlich einfacher möglich als eine Dorsalextionsbewegung über die Nullstellung hinaus.

Das führt, wie oben bereits beschrieben, dazu, dass nach dem Aufsetzen des Prothesenfußes das Gewicht einfach auf den Prothesenfuß übertragen werden kann. Sobald das Gewicht vollständig auf den Prothesenfuß übertragen ist, muss gemäß einer bevorzugten Ausführungsform das Knöchel-Drehmoment den Freigabe-Schwellenwert überschreiten, damit es zu einer weiteren Dorsalextensionsbewegung kommt. Es ergibt sich so ein natürlicher Bewegungsablauf bei geringer Stolpergefahr.

Im Rahmen der vorliegenden Beschreibung wird unter dem Strömungswiderstand der Quotient aus Druck und Durchfluss verstanden, beispielsweise bei einem Durchfluss von 1 Milliliter pro Sekunde.

Vorzugsweise ist die Überbrückungsleitung so mit dem Hydraulikzylinder verbunden, dass eine Abzapfstelle einer Abzapföffnung entlang einer Längsachse des Hydraulikzylinders zur Einstellung der Nullstellung veränderbar ist.

Die Abzapföffnung ist diejenige Öffnung, aus der das Hydraulikfluid aus dem Hydraulikzylinder in die Überbrückungsleitung gelangen kann. Sobald der Kolben des Hydraulikzylinders die Abzapfstelle passiert hat, hemmt die Hemmvorrichtung eine weitere Bewegung des Kolbens oder unterbindet sie, insbesondere bis das Knöchel-Drehmoment den Freigabe-Schwellenwert überschritten hat.

Gemäß einer bevorzugten Ausführungsform weist die erste Fluidleitung eine Rückschlagarmatur auf, die ausgebildet ist zum Unterbinden einer Dorsalextensionsbewegung des Fußteils über die Nullstellung hinaus, solange das Knöchel-Drehmoment höchstens nicht oberhalb des Freigabe-Schwellenwerts liegt, und zum Freigeben der Dorsalextensionsbewegung über die Nullstellung hinaus, wenn das Knöchel-Drehmoment oberhalb des Freigabe-Schwellenwerts liegt.

Eine derartige Rückschlagarmatur ist robust, langlebig und leise und zudem einfach herzustellen. Unter einer Rückschlagarmatur wird eine Komponente verstanden, die einen Fluss des Hydraulikfluids lediglich in eine Durchflussrichtung erlaubt. Beispielsweise handelt es sich bei der Rückschlagarmatur um ein Rückschlagventil oder ein Folgeventil.

Günstig ist es, wenn die Hemmvorrichtung eine zweite Fluidleitung aufweist, durch die Hydraulikfluid fließt, wenn das Fußteil eine Plantarflexionsbewegung ausführt, wobei die zweite Fluidleitung einen zweiten Strömungswiderstand hat, der kleiner ist als der erste Strömungswiderstand. Hierunter wird insbesondere verstanden, dass der zweite Strömungswiderstand höchstens ein Drittel, insbesondere höchstens ein Fünftel, des ersten Strömungswiderstands der ersten Fluidleitung beträgt. Das führt dazu, dass eine Plantarflexionsbewegung, wie sie vom Fußteil zu Beginn des Gangzyklus beim Aufsetzen mit der Ferse ausgeführt wird, vergleichsweise einfach möglich ist. Es ergibt sich dadurch ein mit dem natürlichen Gangbild.

Gemäß einer bevorzugten Ausführungsform besitzt die Freigabevorrichtung einen zweiten Hydraulikzylinder, der so mit der Hemmvorrichtung verbunden ist, dass eine Dorsalextensionsbewegung des Fußteils über die Nullstellung hinaus dazu führt, dass Hydraulikfluid aus dem zweiten Hydraulikzylinder durch die erste Fluidleitung in den ersten Hydraulikzylinder fließt, eine Dorsalextensionsbewegung zwischen der Maximal-Plantarflexionsstellung und der Nullstellung dazu führt, dass das Hydraulikfluid durch die Überbrückungsleitung fließt und dass eine Plantarflexionsbewegung dazu führt, dass das Hydraulikfluid aus dem ersten Hydraulikzylinder durch die zweite Fluidleitung in den zweiten Hydraulikzylinder fließt.

Es sei darauf hingewiesen, dass die erste Fluidleitung, die zweite Fluidleitung und/oder die Überbrückungsleitung gemeinsame Leitungsabschnitte aufweisen können.

Alternativ ist der Hydraulikzylinder doppeltwirkend ausgebildet und so mit der Hemmvorrichtung verbunden, dass eine Dorsalextensionsbewegung des Fußteils über die Nullstellung hinaus dazu führt, dass das Hydraulikfluid aus einer Auslassöffnung, die an einem Auslassende des Hydraulikzylinders angeordnet ist, durch eine erste Fluidleitung und gegebenenfalls durch eine Einlassöffnung, die an einem dem Auslassende gegenüberliegenden Einlassende angeordnet ist, zurück in den Hydraulikzylinder fließt, dass eine Dorsalextensionsbewegung zwischen der Maximal-Plantarflexionsbewegung und der Nullstellung dazu führt, dass das Hydraulikfluid durch die Abzapföffnung und die Überbrückungsleitung fließt und dass eine Plantarflexionsbewegung dazu führt, dass das Hydraulikfluid durch die zweite Fluidleitung fließt. Es ist zwar denkbar und erfindungsgemäß, dass neben einem doppeltwirkenden Hydraulikzylinder ein zweiter Hydraulikzylinder vorhanden ist, das führt aber zu einem erhöhten Fertigungsaufwand, der in den meisten Fällen unerwünscht ist.

Gemäß einer bevorzugten Ausführungsform umfasst die Freigabevorrichtung eine Reibbremse und ein mechanisches Rückschlagelement, wobei die Reibbremse und das Rückschlagelement so angeordnet sind, dass eine Dorsalextensionsbewegung des Fußteils stärker gebremst wird als eine Plantarflexionsbewegung. Beispielsweise ist die Reibbremse mittels einer Feder auf eine Hemmstruktur vorgespannt.

Günstig ist es, wenn die Hemmvorrichtung einen Zapfen, der starr mit dem Fußteil verbunden ist, einen Außenkranz, der starr mit dem Unterschenkel-Anschlussteil verbunden ist, und einen Zwischenring, der relativ zum Zapfen und relativ zum Außenkranz drehbar gelagert ist, aufweist, wobei das mechanische Rückschlagelement so angeordnet ist, dass sich der Zwischenring nur bei einer Dorsalextensionsbewegung des Fußteils relativ zum Zapfen oder relativ zum Unterschenkel-Anschlussteil bewegt. Auf diese Weise wird erreicht, dass die Reibbremse, die gemäß einer bevorzugten Ausführungsform eine Reibkraft zwischen dem Zwischenring und entweder dem Unterschenkel-Anschlussteil oder dem Fußteil ausübt, diese Reibkraft nur dann aufbringt, wenn das Fußteil eine Dorsalextensionsbewegung durchführt.

Im Folgenden wird die Erfindung anhand der beigefügten Zeichnungen näher erläutert. Dabei zeigt

Figur 1      eine erfindungsgemäße Fußprothese in dorsalextendierter Stellung am Ende der Schwungphase eines Gangzyklus,

Figur 2a      ein Schaltbild der Freigabevorrichtung der Fußprothese gemäß Figur 1,

Figur 2b      eine Detailansicht des zweiten Hydraulikzylinders,

Figur 3      eine schematische Darstellung des Gangzyklus zur Erläuterung der Wirkungsweise der erfindungsgemäßen Fußprothese,

Figur 4    die Fußprothese in ihrer Nullstellung, die auch als Neutralposition bezeichnet werden könnte,

Figur 5    die Fußprothese in Nullstellung, wenn sie in einem Schuh mit einer höheren Absatzhöhe verwendet wird,

Figur 6    die Fußprothese in ihrer Nullstellung, wenn sie in einem Schuh mit einer niedrigeren Absatzhöhe verwendet wird, und

Figur 7    eine perspektivische Ansicht einer Fußprothese gemäß einer zweiten Ausführungsform der Erfindung und

Figur 8    ein Schaltbild einer Freigabevorrichtung für eine Fußprothese gemäß einer zweiten Ausführungsform der Erfindung.

Figur 9    zeigt eine erfindungsgemäße Fußprothese gemäß einer zweiten Ausführungsform der Erfindung.

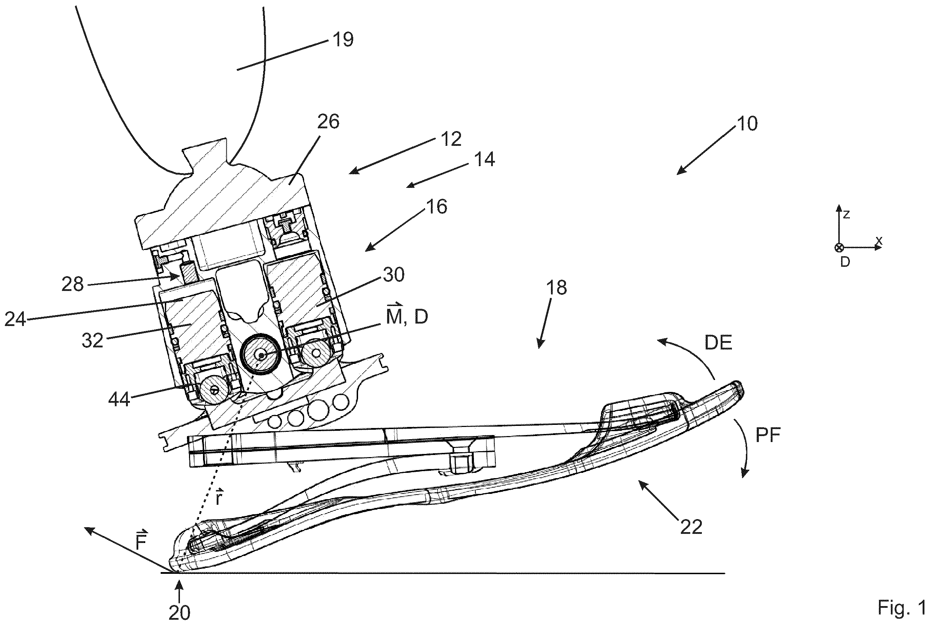

Figur 1 zeigt eine erfindungsgemäße Fußprothese 10, die ein Unterschenkel-Anschlussteil 12, eine Freigabevorrichtung 16 und ein Fußteil 18 aufweist. Das Verbindungselement 14 wirkt als Gelenk, sodass das Fußteil 18 relativ zum Unterschenkel-Anschlussteil 12 in eine Dorsalextensionsrichtung DE schwenkbar ist, die durch den Pfeil DE angedeutet ist. Das Fußteil 18 kann zudem in eine der Dorsalextensionsrichtung DE entgegengesetzte Plantarflexionsrichtung PF geschwenkt werden. Das Unterschenkel-Anschlussteil 12 ist an einem künstlichen Unterschenkel (19) befestigt.

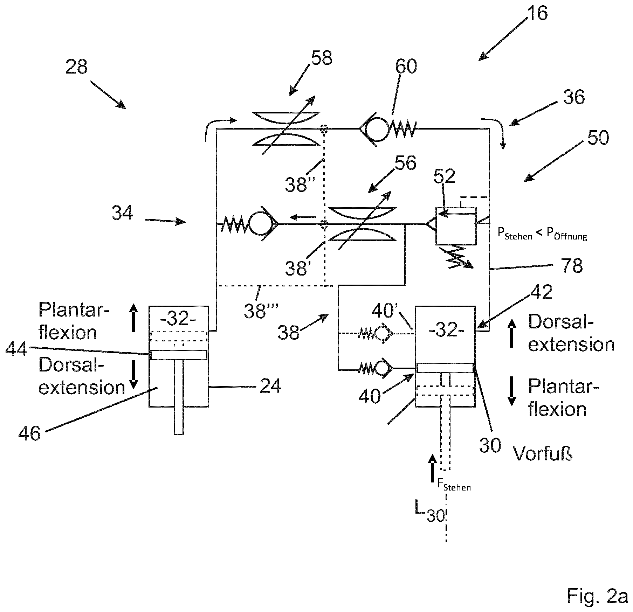

Das Fußteil 18 besitzt einen Fersenabschnitt 20 und einen Ballenabschnitt 22. Die Freigabevorrichtung 16 dient dazu, die Bewegung des Fußteils 18 relativ zum Unterschenkel-Anschlussteil 12 je nach Winkelstellung zu hemmen oder leicht zu ermöglichen. Dazu besitzt die Freigabevorrichtung 16 einen ersten Hydraulikzylinder 24, der einerseits über ein Koppelelement 26 mit dem Fußteil 18 und andererseits mit dem Unterschenkel-Anschlussteil 12 verbunden ist. Zudem besitzt die Freigabevorrichtung 16 eine hydraulische Hemmvorrichtung 28, die in Figur 1 nur in groben Zügen zu erkennen ist und in Figur 2a näher erläutert wird.

Die Freigabevorrichtung 16 besitzt zudem einen zweiten Hydraulikzylinder 30, der so mit dem ersten Hydraulikzylinder 24 verbunden ist, dass ein Hydraulikfluid 32, im vorliegenden Fall eine Hydraulikflüssigkeit, beispielsweise in Form von Öl, die aus einem der Hydraulikzylinder 24, 30 verdrängt wird, in den jeweils anderen Hydraulikzylinder 30, 24 fließt.

Figur 2a zeigt ein Schaltbild der Freigabevorrichtung 16 mit dem ersten Hydraulikzylinder 24, dem zweiten Hydraulikzylinder 30 und der Hemmvorrichtung 28, die hier durch eine Anordnung hydraulischer Komponenten gebildet ist. Die Hemmvorrichtung 28 besitzt eine erste Fluidleitung 34 und eine zweite Fluidleitung 36, die jeweils beide die Hydraulikzylinder 24, 30 miteinander verbinden. Zudem besitzt die Freigabevorrichtung 16 eine Überbrückungsleitung 38, die in einer Abzapföffnung 40 mit einem Zylinderinnenraum 42 des zweiten Hydraulikzylinders 30 in Verbindung steht. Die Abzapfstelle entlang einer Längsachse $L_{32}$ entlang des zweiten Hydraulikzylinders 30 ist einstellbar, wie weiter unten erläutert wird.

Führt das Fußteil 18 (vgl. Figur 1) eine Dorsalextensionsbewegung durch, so bewegt sich ein erster Kolben 44 des ersten Hydraulikzylinders 24 so, dass sich ein Zylinderinnenraum 46 vergrößert. Zudem bewegt sich ein zweiter Kolben 48 des zweiten Hydraulikzylinders 30 in den Zylinderinnenraum 42 hinein.

Befindet sich das Fußteil 18 (vgl. Figur 1) relativ zum Unterschenkel-Anschlussteil 12 in seiner Nullstellung, so ist der Kolben 48 auf Höhe der Abzapföffnung 40. Diese Situation ist in Figur 2a gezeigt. Eine weitere Dorsalextensionsbewegung führt dazu, dass das Hydraulikfluid 32 durch die erste Fluidleitung 34 in den ersten Hydraulikzylinder 24 fließt. Dazu passiert das Hydraulikfluid ausschließlich die erste Fluidleitung 34. Diese umfasst eine Rückschlagarmatur 50, die im vorliegenden Fall ein Folgeventil 52 und ein Rückschlagventil 54 aufweist. Zudem enthält die erste Fluidleitung 34 eine Drossel 56. Es ist möglich, nicht aber notwendig, dass die Drossel 56 als eigenständiges Bauteil ausgebildet ist. Es ist auch möglich, dass Teile der ersten Fluidleitung 34 einen so kleinen Querschnitt aufweisen, dass eine Drosselwirkung erzielt wird. Gemäß einer bevorzugten Ausführungsform ist die Drossel 56 verstellbar ausgebildet, sodass ein Strömungswiderstand, den die erste Fluidleitung dem Hydraulikfluid entgegensetzt, einstellbar ist.

Befindet sich der zweite Kolben 48 vor der Abzapföffnung 40, wie es durch den gestrichelten Kolben 48' angedeutet ist, so fließt das Hydraulikfluid durch die Überbrückungsleitung 38. Es ist möglich, dass die Überbrückungsleitung 38 wie eingezeichnet bezüglich einer Strömungsrichtung vor der Drossel 56 in die erste Fluidleitung 34 einmündet.

Alternativ ist es möglich, dass die Überbrückungsleitung 38 in Strömungsrichtung hinter der Drossel 56 einmündet, was als Überbrückungsleitung 38' gestrichelt eingezeichnet ist. Die Überbrückungsleitung kann zudem in die zweite Fluidleitung 36 münden, was als 38" gekennzeichnet ist. Es ist zudem möglich, dass vor der entsprechenden Einmündungsstelle eine weitere Drossel angeordnet ist, die nicht eingezeichnet ist. Gemäß einer weiteren Ausführungsform mündet die Überbrückungsleitung 38''' direkt in den ersten Hydraulikzylinder 24. In diesem Fall ist es günstig, wenn die Überbrückungsleitung 38 eine eigene Rückschlagarmatur und gegebenenfalls eine eigene Drossel aufweist.

Führt das Fußteil 18 eine Plantarflexionsbewegung aus, so bewegt sich der erste Kolben 44 in den Zylinderinnenraum des ersten Hydraulikzylinders 24 und das Hydraulikfluid fließt nur durch die zweite Fluidleitung 36 in den zweiten Hydraulikzylinder 30. Die zweite Fluidleitung 36 besitzt eine zweite Drossel 58 und eine zweite Rückschlagarmatur 60. Es ist möglich, dass die Überbrückungsleitung 38 wie strichpunktiert eingezeichnet ist, in die zweite Fluidleitung 36 mündet.

Die erste Rückschlagarmatur 50 besitzt einen Öffnungsdruck $p_{\text{öffnung}}$ und öffnet erst, wenn ein Druck $p_{42}$ im Zylinderinnenraum 42 des zweiten Hydraulikzylinders 30 überschritten ist. Der Öffnungsdruck $p_{\text{öffnung}}$ wird genau dann erreicht, wenn die Projektion des Knöchel-Drehmoments $\vec{M}$ auf eine Drehachse D, um die das Fußteil 18 relativ zum Unterschenkel-Anschlussteil 12 schwenkt, einen Freigabe-Schwellenwert überschreitet. Wirkt im Ballenabschnitt 22 eine Kraft, die das Fußteil 18 in Dorsalextensionsrichtung DE drückt, so wird diese Drehmoment als positiv angesehen. Wirkt das Fußteil 18 hingegen eine Kraft, die zu einer Plantarflexionsbewegung des Fußteils führt, so wird dieses Knöchel-Drehmoment als negativ betrachtet.

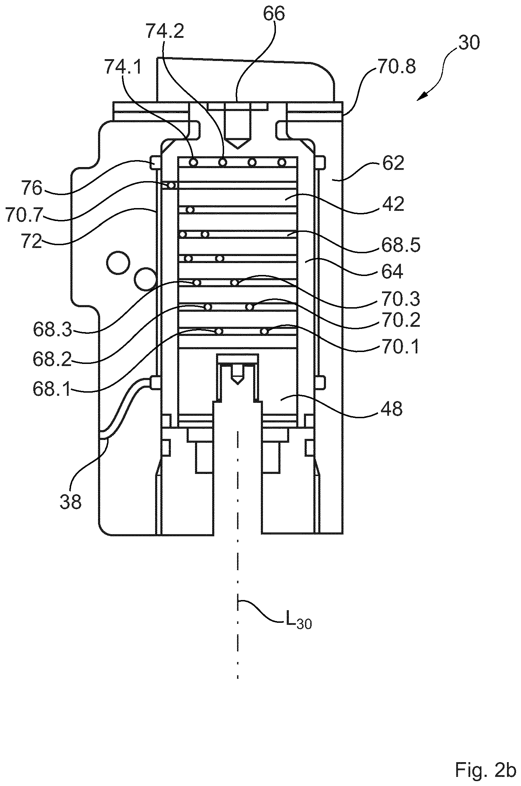

Figur 2b zeigt schematisch, dass der zweite Hydraulikzylinder 30 einen Grundkörper 62 aufweist, in dem eine Hülse 64 drehbar gelagert ist. Die Hülse 64 besitzt eine Koppelstruktur 66, in die mit einem geeigneten Werkzeug eingegriffen werden kann.

Die Innenwandung der Hülse 64 bildet die Zylinderinnenwand des zweiten Hydraulikzylinders 30. In die Hülse 64 sind mehrere Nuten 68.1, 68.2, ... eingebracht. Zudem besitzt die Hülse 64 mehrere Bohrungen 70,1, 70.2, .... Im vorliegenden Fall besitzt, was eine bevorzugten Ausführungsform darstellt, jede Nut 68.i (i=1,2,...) genau eine Bohrung 70.i. Die Bohrungen 70.i sind winkelversetzt um die Längsachse $L_{30}$ angeordnet. Die Bohrung 70.7 liegt im vorliegenden Fall in der Schnittebene der Zeichnung. Es ist zu erkennen, dass die Bohrung 70.7 mit einem Rückführkanal 72 verbunden ist. Hydraulikfluid kann daher durch die Bohrung 70.7 und nur durch diese aus dem Zylinderinnenraum 42 nach außen gelangen.

Die Hülse 64 ist gegenüber dem Grundkörper 62 abgedichtet und der Rückführkanal 72 ist so um eine Außenkontur der Hülse 64 herum angeordnet, dass stets nur eine Bohrung, hier 70.7, mit dem Rückführkanal 72 verbunden ist. Insbesondere ist die Breite des Rückführkanals 72 in Umfangsrichtung der Hülse 64 kleiner als die Bogenlänge desjenigen Winkelabstands, um den die Bohrungen 70 gegeneinander versetzt sind. Der Rückführkanal 72 ist Teil der Überbrückungsleitung 38.

Die dem Kolben 48 am weitesten beabstandete Nut, im vorliegenden Fall die Nut 70.8, besitzt mehrere Bohrungen 74.1, 74.2, ..., durch die Hydraulikfluid bei jeder Drehstellung der Hülse 64 in einen Ringkanal 76 gelangen kann. Der Ringkanal ist Teil einer Anschlussleitung 78, die sowohl Teil der ersten Fluidleitung 34 (vgl. Figur 2a) als auch der zweiten Fluidleitung 36 ist.

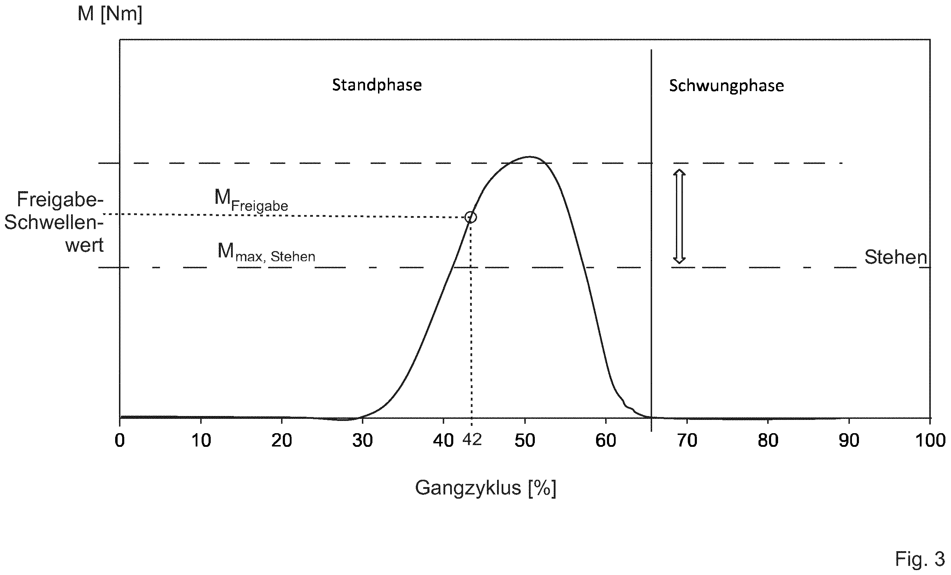

Figur 3 zeigt schematisch den Verlauf des Knöchel-Drehmoments M als skalare Größe vom Gangzyklus.

Es ist zu erkennen, dass im Stehen ein Knöchel-Drehmoment M einen Maximalwert $M_{\text{max,Stehen}}$ nicht überschreitet. Beim Gehen hingegen übersteigt das Knöchel-Drehmoment M den Wert $M_{\text{max,Stehen}}$. Solange der Freigabe-Schwellenwert $M_{\text{Freigabe}}$ nicht überschritten ist, sperrt die Freigabevorrichtung 16 (vgl. Figur 1) die Bewegung des Fußteils 18.

Ist der Freigabe-Schwellenwert $M_{\text{Freigabe}}$ überschritten, gibt die Freigabevorrichtung 16 die Dorsalextensionsbewegung des Fußteils 18 frei. Nach Ende der Schwungphase und erneutem Beginn des Gangzyklus wird das Knöchel-Drehmoment M zeitweise negativ. Das ist dann der Fall, wenn der Fersenabschnitt 20 den Boden berührt. Die Freigabevorrichtung 16 erlaubt in diesem Fall eine Plantarflexionsbewegung des Fußteils 18 (vgl. Figur 1).

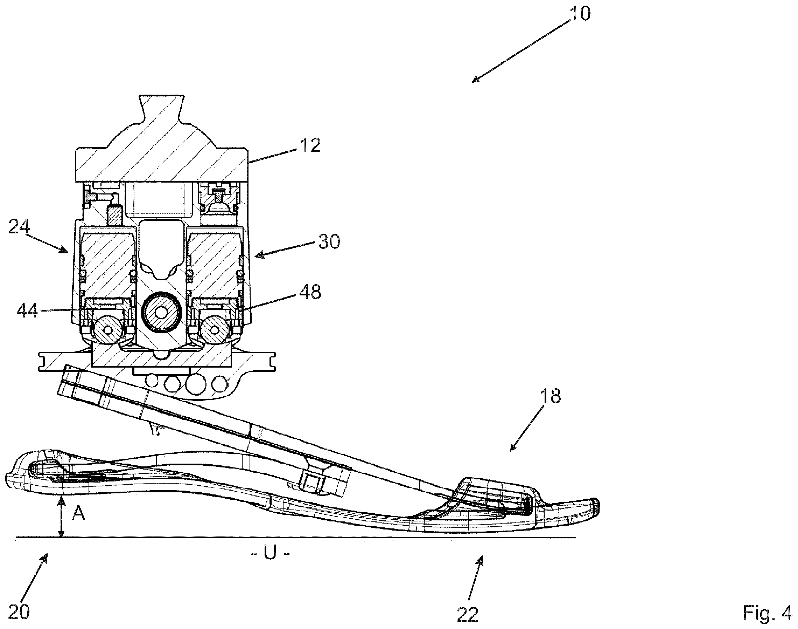

Figur 4 zeigt die Fußprothese 10, bei der das Fußteil 18 relativ zum Unterschenkel-Anschlussteil 12 in seiner Nullstellung ist, die auch als Neutralposition bezeichnet werden könnte. In dieser Position steht der Patient. Der Fersenabschnitt 20 ist um eine Absatzhöhe A vom Untergrund U beabstandet. Der Schuh, der die gleiche Absatzhöhe A hat, ist nicht mit eingezeichnet. In der in Figur 4 gezeigten Position liegen auf dem Fersenabschnitt 20 und dem Ballenabschnitt 22 ungefähr gleich große Kräfte, die über den nicht eingezeichneten Schuh in den Untergrund U eingeleitet werden. Es ist zu erkennen, dass die Kolben 44, 48 der beiden Hydraulikzylinder 24,30 auf ungefähr gleicher Höhe angeordnet sind, was eine bevorzugte Ausführungsform darstellt, nicht aber notwendig ist.

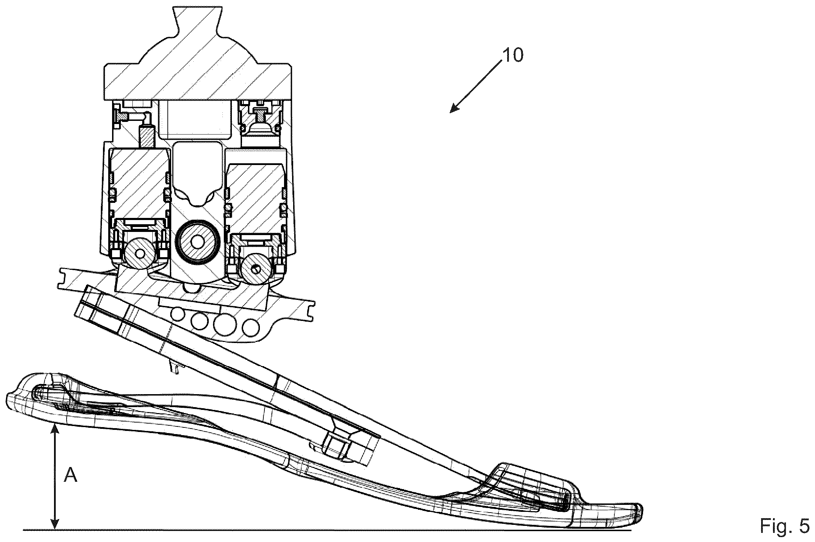

Figur 5 zeigt die Fußprothese 10 in der Nullstellung bei Verwendung eines Schuhs mit einer größeren Absatzhöhe A. Um den zusätzlichen Winkelversatz des Fußteils 18 im Vergleich zu der Nullstellung bei mittlerer Absatzhöhe, wie sie in Figur 4 gezeigt ist, herzustellen, wird die Abzapföffnung 40 an eine andere Abzapfstelle verlegt, wie dies oben in Figuren 2a und 2b beschrieben ist. Da bei der in Figur 5 gezeigten erhöhten Absatzhöhe das maximale Knöchel-Drehmoment M kleiner ist als für den in Figur 4 gezeigten Fall, ist es vorteilhaft, wenn der Öffnungsdruck $p_{\text{öffnung}}$ der Rückschlagarmatur 50 einstellbar ist.

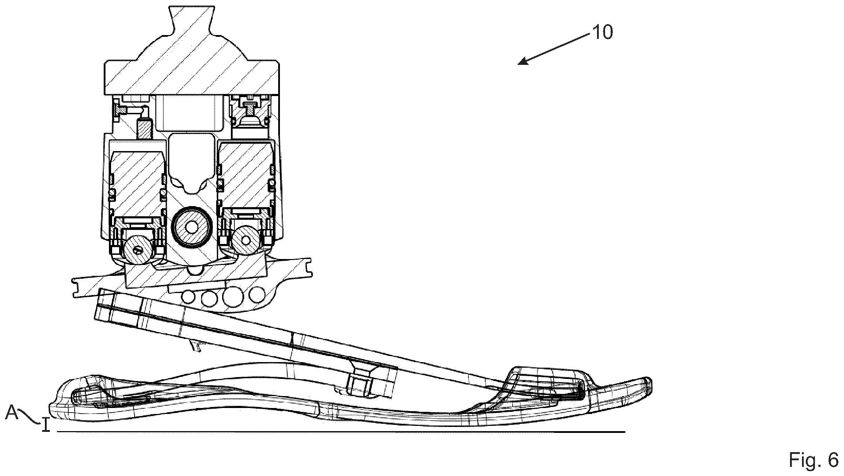

Figur 6 zeigt die Fußprothese 10 bei Verwendung eines Schuhs mit einer geringeren Absatzhöhe als in Figur 4. Auch in diesem Fall wird der im Vergleich zur Darstellung in Figur 4 eintretende Winkelversatz durch Verstellen des Abzapforts der Abzapföffnung 40 kompensiert.

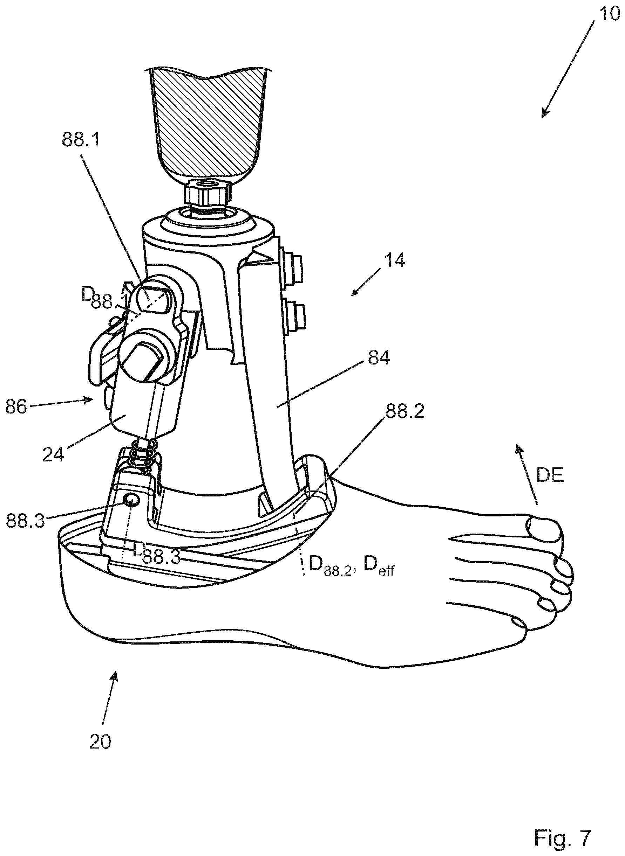

Figur 7 zeigt eine zweite Ausführungsform einer erfindungsgemäßen Fußprothese 10. Das Fußteil 18 umfasst eine Fußkosmetik 80, die der Prothese 10 ein natürliches Aussehen verleiht. Die Fußkosmetik 80 ist an einer Standplatte 82 befestigt, die ebenfalls Teil des Fußteils 18 ist. Das Verbindungselement 14 umfasst einen starren Arm 84, der am Fußteil 18 angelenkt ist. Die Freigabevorrichtung 16 bildet einen zweiten, längenveränderlichen Arm 86, der am Fersenabschnitt 20 mit dem Fußteil 18 verbunden ist.

Das Verbindungselement 14 weist drei Teil-Drehgelenke, 88.1, 88.2, 88.3, die alle drei Drehgelenke sind und jeweilige Drehachsen $D_{88.1}$, $D_{88.2}$ und $D_{88.3}$ aufweisen. Bei Bewegung der Prothese 10 dreht sich das Fußteil 18 relativ zum Unterschenkel-Anschlussteil 12 um eine Drehachse $D_{eff}=D_{88.2}$. Der Hydraulikzylinder 24 ist mit seiner Kolbenstange am Teilgelenk 88.2 und mit seinem Zylindergehäuse am Unterschenkel-Anschlussteil 12 angelenkt.

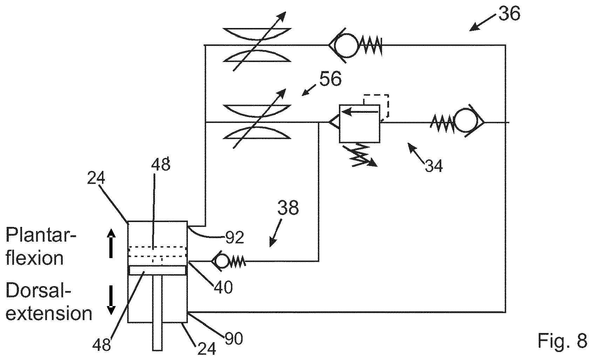

Figur 8 zeigt das hydraulische Ersatzschaltbild für die Ausführungsform gemäß Figur 7. Der Hydraulikzylinder 24 ist doppeltwirkend ausgebildet. Eine Dorsalextensionsbewegung über die Nullstellung hinaus, die durch den Kolben 48 in durchgezogener Linie angedeutet ist, führt dazu, dass das Hydraulikfluid aus einer Auslassöffnung 90, durch die erste Fluidleitung 34 und durch eine Einlassöffnung 92 zurück in den Hydraulikzylinder 24 fließt.

Eine Dorsalextensionsbewegung zwischen der Maximal-Plantarflexionsstellung, in der das Fußteil 18 (vgl. Figur 7) maximal plantar reflektiert relativ zum Unterschenkel-Anschlussteil 12 angeordnet ist, und der Nullstellung führt dazu, dass das Hydraulikfluid durch die Abzapföffnung (40) und die Überbrückungsleitung 38 sowie durch die Drossel 56 und die Einlassöffnung 72 zurück in den Hydraulikzylinder 24 fließt. Eine Plantarflexionsbewegung führt dazu, dass das Hydraulikfluid durch die zweite Fluidleitung 36 und die Auslassöffnung 90 und /oder durch die Überbrückungsleitung 38 fließt.

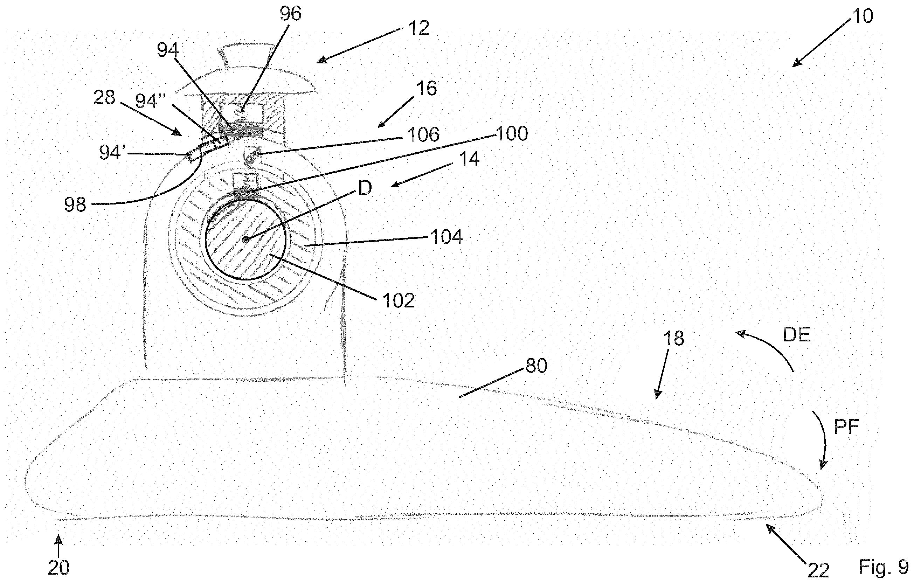

Figur 9 zeigt eine nicht mehr von der Erfindung umfasste Ausführungsform. Die Hemmvorrichtung 28 umfasst ein Gleitelement 94, das durch ein Vorspannelement 96 auf eine Hemmstruktur 98 zu gespannt wird. Die Hemmstruktur 98 ist im vorliegenden Fall am Fußteil 18 ausgebildet. Befindet sich das Gleitelement 94, wie gestrichelt angedeutet und mit der Bezugszahl 94' versehen, in einem ersten Abschnitt der Hemmstruktur 98, so setzt es einer Dorsalextensionsbewegung nur einen geringen Widerstand entgegen. Dieser geringe Widerstand liegt an, wenn das Fußteil 18 relativ zum Unterschenkel-Anschlussteil 12 sich zwischen einer Maximal-Plantarflexionsstellung und der Nullstellung befindet. Das Gleitelement 94 fungiert zudem als Reibelement, sodass zum Reiben des Gleitelements 94 an Hemmstruktur 98 eine Reibkraft überwunden werden muss.

Befindet sich das Fußteil 18 in seiner Nullstellung, so befindet sich das Gleitelement 94 an einer Stelle, an der eine weitere Bewegung nur gegen die Federkraft des Vorspannelements 96 möglich ist. Das ist nur möglich, wenn am Fußteil 18 ein Knöchel-Drehmoment anliegt, das den Freigabe-Schwellenwert überschreitet. Figur 9 zeigt die Fußprothese 10 in ihrer Maximal-Dorsalextensionsstellung.

Die Hemmvorrichtung 28 umfasst eine Reibbremse 100, mittels der eine Drehbewegung zwischen Fußteil 18 und Unterschenkel-Anschlussteil 12 bremsbar ist. Im vorliegenden Fall ist die Reibbremse 100 zum Bremsen einer Bewegung eines Zapfens 102, der starr mit dem Fußteil 18 verbunden ist, und einem Zwischenring 104, der auch als Kupplungsring bezeichnet werden könnte, ausgebildet. Der Zwischenring 104 ist drehbar sowohl relativ zum Fußteil 18 als auch zum Unterschenkel-Anschlussteil 12 ausgebildet.

Die Freigabevorrichtung 16 umfasst ein mechanisches Rückschlagelement 106, das so mit dem Fußteil 18 verbunden ist, dass eine Dorsal-Extensionsbewegung des Fußteils 18 zu keiner Relativbewegung zwischen dem Fußteil 18 und dem Zwischenring 104 führt. Hingegen ermöglicht das Rückschlagelement 106 eine Bewegung zwischen dem Zwischenring 104 und dem Fußteil 18, wenn das Fußteil 18 eine Plantarflexionsbewegung ausführt. Auf diese Weise wird erreicht, dass die Reibbremse 100 lediglich eine Dorsalextensionsbewegung des Fußteils bremst, nicht aber eine Plantarflexionsbewegung. Durch Vorspannen der Feder 96 kann der Freigabe-Schwellenwerts $M_{Freigabe}$ eingestellt werden.

Es ist möglich, dass die Hemmstruktur 98 relativ zum Fußteil 18 in einer bezüglich der Drehachse D radialen Richtung lösbar befestigt ist. Beispielsweise ist die Hemmstruktur 98 an einem relativ zum Fußteil 18 festlegbar bewegbaren Einstellteil ausgebildet ist. In diesem Fall kann dieses Einstellteil zunächst vom Fußteil 18 gelöst, dann relativ zum Fußteil 18 um die Drehachse D geschwenkt und danach wieder am Fußteil 18 befestigt werden. Auf diese Weise verändert sich die Winkelstellung, in der das Fußteil 18 in der Nullstellung ist. So kann die Nullstellung eingestellt werden, ab der die Dorsalextensionsbewegung stärker gehemmt wird.

## Bezugszeichenliste

| | | | |
|---|---|---|---|
| 10 | Fußprothese | 66 | Koppelstruktur |
| 12 | Unterschenkel-Anschlussteil | 68 | Nut |
| 14 | Verbindungselement | | |
| 16 | Freigabevorrichtung | 70 | Bohrung |
| 18 | Fußteil | 72 | Rückführkanal |
| 19 | Unterschenkel | 74 | Bohrung |
| | | 76 | Ringkanal |
| 20 | Fersenabschnitt | 78 | Anschlussleitung |
| 22 | Ballenabschnitt | | |
| 24 | Hydraulikzylinder | 80 | Fußkosmetik |
| 26 | Koppelelement | 82 | Standplatte |

(fortgesetzt)

| 28 | Hemmvorrichtung | 84 | starrer Arm |
|---|---|---|---|
| | | 86 | Teil-Drehgelenk |
| 30 | zweiter Hydraulikzylinder | | |
| 32 | Hydraulikfluid | 90 | Auslassöffnung |
| 34 | erste Fluidleitung | 92 | Einlassöffnung |
| 36 | zweite Fluidleitung | 94 | Gleitelement |
| 38 | Überbrückungsleitung | 96 | Vorspannelement |
| | | 98 | Hemmstruktur |
| 40 | Abzapföffnung | | |
| 42 | Zylinderinnenraum | 100 | Reibbremse |
| 44 | erste Kolben | 102 | Zapfen |
| 46 | Zylinderinnenraum | 104 | Zwischenring |
| 48 | zweiter Kolben | 106 | Rückschlagelement |
| 50 | Rückschlagarmatur | A | Absatzhöhe |
| 52 | Folgeventil | D | Drehachse |
| 54 | Rückschlagventil | DE | Dorsalextensionsrichtung |
| 56 | Drossel | $L_{30}$ | Längsachse |
| 58 | zweite Drossel | $M_{Freigabe}$ | Freigabe-Schwellenwert |
| | | $\vec{M}$ | Knöchel-Drehmoment |
| 60 | zweite Rückschlagarmatur | $p_{\text{Öffnung}}$ | Öffnungsdruck |
| 62 | Grundkörper | PF | Plantarflexionsrichtung |
| 64 | Hülse | U | Untergrund |

## Patentansprüche

1. Fußprothese mit

   (a) einem Unterschenkel-Anschlussteil (12),
   (b) einem Fußteil (18),
   (c) einem Verbindungselement (14) mit Gelenkfunktion, das das Unterschenkel-Anschlussteil (12) mit dem Fußteil (18) verbindet, und
   (d) einer Freigabevorrichtung (16),
   mittels der eine Bewegung des Fußteils (18) relativ zum Unterschenkel-Anschlussteil (12) hemmbar und enthemmbar ist,
   die eine passive Hemmvorrichtung (28) besitzt, und die einen Hydraulikzylinder (24), der mit dem Fußteil (18) und dem Unterschenkel-Anschlussteil (12) verbunden ist, aufweist,
   **dadurch gekennzeichnet, dass**
   (e) die Hemmvorrichtung (28) so mit dem Hydraulikzylinder (24) verbunden ist, dass

   ($e_1$) eine Dorsalextensionsbewegung des Fußteils (18) relativ zum Unterschenkel-Anschlussteil (12) in einem Winkelbereich von einer Maximal-Plantarflexionsstellung bis zu einer Nullstellung weniger stark gehemmt wird als eine Dorsalextensionsbewegung des Fußteils (18) aus der Nullstellung, und
   ($e_2$) eine Dorsalextensionsbewegung über die Nullstellung hinaus möglich ist.

2. Fußprothese nach Anspruch 1, **dadurch gekennzeichnet, dass**
   die Freigabevorrichtung (16) so ausgebildet ist, dass sie die Dorsalextensionsbewegung des Fußteils (18) über die Nullstellung hinaus dann und nur dann freigibt, wenn ein Knöchel-Drehmoment ($\vec{M}$), das am Fußteil (18) anliegt, oberhalb eines vorgegebenen Freigabe-Schwellenwerts ($M_{Freigabe}$) liegt.

3. Fußprothese nach einem der vorstehenden Ansprüche, **dadurch gekennzeichnet, dass** die Freigabevorrichtung (16) so ausgebildet ist, dass die Nullstellung einstellbar ist.

4. Fußprothese nach einem der voranstehenden Ansprüche, **dadurch gekennzeichnet, dass** die Hemmvorrichtung (28)

- eine erste Fluidleitung (34), durch die Hydraulikfluid (32) fließt, wenn das Fußteil (18) eine Dorsalextensionsbewegung über die Nullstellung hinaus ausführt, wobei die erste Fluidleitung (34) einen ersten Strömungswiderstand hat, und
- eine Überbrückungsleitung (38), durch die das Hydraulikfluid (32) fließt, wenn das Fußteil (18) eine Dorsalextensionsbewegung zwischen der Maximal-Plantarflexionsstellung und der Nullstellung ausführt,
aufweist,
- wobei die Überbrückungsleitung (38) einen Überbrückungs-Strömungswiderstand hat, der kleiner ist als der erste Strömungswiderstand.

5. Fußprothese nach Anspruch 4, **dadurch gekennzeichnet, dass** die erste Fluidleitung (34) eine Rückschlagarmatur (50) aufweist, die angeordnet ist zum

- Unterbinden einer Dorsalextensionsbewegung über die Nullstellung hinaus, solange das Knöchel-Drehmoment (M) nicht oberhalb des Freigabe-Schwellenwerts ($M_{Freigabe}$) liegt, und zum
- Freigeben der Dorsalextensionsbewegung über die Nullstellung hinaus, wenn das Knöchel-Drehmoment (M) oberhalb des Freigabe-Schwellenwerts liegt.

6. Fußprothese nach Anspruch 4 oder 5, **dadurch gekennzeichnet, dass** die Hemmvorrichtung (28)

eine zweite Fluidleitung (36) aufweist, durch die Hydraulikfluid (32) fließt, wenn das Fußteil (18) eine Plantarflexionsbewegung ausführt,
wobei die zweite Fluidleitung (36) einen zweiten Strömungswiderstand hat, der kleiner ist als der erste Strömungswiderstand.

7. Fußprothese nach Anspruch 6, **dadurch gekennzeichnet, dass** die Freigabevorrichtung (16) einen zweiten Hydraulikzylinder (30) aufweist, der so mit der Hemmvorrichtung (28) verbunden ist, dass

- eine Dorsalextensionsbewegung über die Nullstellung hinaus dazu führt, dass Hydraulikfluid (32) aus dem zweiten Hydraulikzylinder (30) durch die erste Fluidleitung (34) in den ersten Hydraulikzylinder (24) fließt,
- eine Dorsalextensionsbewegung zwischen der Maximal-Plantarflexionsstellung und der Nullstellung dazu führt, dass das Hydraulikfluid (32) aus dem zweiten Hydraulikzylinder (30) durch die Überbrückungsleitung (38) in den ersten Hydraulikzylinder (24) fließt und
- eine Plantarflexionsbewegung dazu führt, dass das Hydraulikfluid (32) aus dem ersten Hydraulikzylinder (24) durch die zweite Fluidleitung (36) in den zweiten Hydraulikzylinder (30) fließt.

8. Fußprothese nach Anspruch 7, **dadurch gekennzeichnet, dass** der Hydraulikzylinder (24) doppelwirkend ausgebildet und so mit der Hemmvorrichtung (28) verbunden ist, dass

- eine Dorsalextensionsbewegung über die Nullstellung hinaus dazu führt, dass das Hydraulikfluid (32) aus einer Auslassöffnung (90), die an einem Auslassende des Hydraulikzylinders (24) angeordnet ist, durch die erste Fluidleitung (34) zurück in den Hydraulikzylinder (24) fließt,
- eine Dorsalextensionsbewegung zwischen der Maximal-Plantarflexionsstellung und der Nullstellung dazu führt, dass das Hydraulikfluid (32) durch eine Abzapföffnung (40) und die Überbrückungsleitung (38) fließt und
- eine Plantarflexionsbewegung dazu führt, dass das Hydraulikfluid (32) durch die zweite Fluidleitung (36) oder die Überbrückungsleitung (38) fließt.

9. Fußprothese nach Anspruch 8, **dadurch gekennzeichnet, dass**
die Überbrückungsleitung (38) so mit dem ersten Hydraulikzylinder (34) oder dem zweiten Hydraulikzylinder (30) verbunden ist, dass eine Abzapfstelle einer Abzapföffnung entlang einer Längsachse (L) des Hydraulikzylinders (24) zum Einstellen der Nullstellung veränderbar ist.

**Claims**

1. A foot prosthesis with

(a) a lower leg connection part (12),

(b) a foot part (18),

(c) a connection element (14) with joint function that connects the lower leg connection part (12) with the foot part (18), and

(d) a release device (16),

by means of which a movement of the foot part (18) relative to the lower leg connection part (12) can be restrained and unrestrained,

which has a passive restraining device (28) and which comprises a hydraulic cylinder (24) that is connected to the foot part (18) and the lower leg connection part (12),

**characterised in that**

(e) the restraining device (28) is connected to the hydraulic cylinder (24) in such a way that

($e_1$) a dorsal extension of the foot part (18) relative to the lower leg connection part (12) in an angular range from a maximum plantar flexion position to a neutral position is less strongly restrained than a dorsal extension movement of the foot part (18) out of of the neutral position, and

($e_2$) a dorsal extension movement beyond the neutral position is possible.

2. The foot prosthesis according to claim 1, **characterised in that**
the release device (16) is designed in such a way that it only releases the dorsal extension movement of the foot part (18) beyond the neutral position when an ankle torque ($\vec{M}$) acting on the foot part (18) is above a predetermined release threshold value ($M_{Freigabe}$).

3. The foot prosthesis according to one of the preceding claims, **characterised in that** the release device (16) is designed such that the neutral position is adjustable.

4. The foot prosthesis according to one of the preceding claims, **characterised in that** the restraining device (28) comprises

- a first fluid line (34) through which hydraulic fluid (32) flows when the foot part (18) executes a dorsal extension movement beyond the neutral position, the first fluid line (34) having a first flow resistance, and
- a bypass line (38) through which the hydraulic fluid (32) flows when the foot part (18) executes a dorsal extension movement between the maximum plantar flexion position and the neutral position,
- the bypass line (38) having a bypass flow resistance that is smaller than the first flow resistance.

5. The foot prosthesis according to claim 4, **characterised in that** the first fluid line (34) comprises a check valve (50) that is arranged to

- prevent a dorsal extension movement beyond the neutral position as long as the ankle torque (M) is not above the release threshold value ($M_{Freigabe}$), and to
- release the dorsal extension movement beyond the neutral position when the ankle torque (M) is above the release threshold value.

6. The foot prosthesis according to claim 4 or 5, **characterised in that** the restraining device (28)

comprises a second fluid line (36) through which hydraulic fluid flows (32) when the foot part (18) executes a plantar flexion movement,
the second fluid line (36) having a second flow resistance that is smaller than the first flow resistance.

7. The foot prosthesis according to claim 6, **characterised in that** the release device (16) comprises a second hydraulic cylinder (30) that is connected to the restraining device (28) in such a way that

- a dorsal extension movement beyond the neutral position causes hydraulic fluid (32) to flow out of the second hydraulic cylinder (30), through the first fluid line (34) and into the first hydraulic cylinder (24),
- a dorsal extension movement between the maximum plantar flexion position and the neutral position causes the hydraulic fluid (32) to flow out of the second hydraulic cylinder (30), through the bypass line (38) and into the first hydraulic cylinder (24), and
- a plantar flexion movement causes the hydraulic fluid (32) to flow out of the first hydraulic cylinder (24), through the second fluid line (36) and into the second hydraulic cylinder (30).

8. The foot prosthesis according to claim 7, **characterised in that** the hydraulic cylinder (24) is designed to have a dual effect and is connected to the restraining device (28) in such a way that

- a dorsal extension movement beyond the neutral position causes hydraulic fluid (32) to flow out of an outlet opening (90), which is arranged at an outlet end of the hydraulic cylinder (24), through the first fluid line (34) and back into the hydraulic cylinder (24),
- a dorsal extension movement between the maximum plantar flexion position and the neutral position causes the hydraulic fluid (32) to flow through a tap opening (40) and the bypass line (38), and
- a plantar flexion movement causes the hydraulic fluid (32) to flow through the second fluid line (36) or the bypass line (38).

9. The foot prosthesis according to claim 8, **characterised in that**
the bypass line (38) is connected to the first hydraulic cylinder (34) or the second hydraulic cylinder (30) in such a way that a tapping point of a tap opening can be altered along a longitudinal axis (L) of the hydraulic cylinder (24) in order to adjust the neutral position.


**Revendications**

1. Pied prothétique comprenant

(a) une partie (12) de raccordement à la jambe,
(b) une partie de pied (18),
(c) un élément de liaison (14) avec fonction d'articulation, qui relie la partie (12) de raccordement à la jambe à la partie de pied (18), et
(d) un dispositif de libération (16),

permettant d'inhiber et de désinhiber un mouvement de la partie de pied (18) par rapport à la partie (12) de raccordement à la jambe, possédant un dispositif d'inhibition passif (28), et
comprenant un vérin hydraulique (24) relié à la partie de pied (18) et à la partie (12) de raccordement à la jambe,

**caractérisé en ce que**
(e) le dispositif d'inhibition (28) est relié au vérin hydraulique (24) de telle sorte que

(e$_1$) un mouvement d'extension dorsale de la partie de pied (18) par rapport à la partie (12) de raccordement à la jambe dans une plage angulaire allant d'une position de flexion plantaire maximale à une position zéro est moins fortement inhibé qu'un mouvement d'extension dorsale de la partie de pied (18) à partir de la position zéro, et
(e$_2$) un mouvement d'extension dorsale au-delà de la position zéro est possible.

2. Pied prothétique selon la revendication 1,
**caractérisé en ce que** le dispositif de libération (16) est conçu de manière à libérer le mouvement d'extension dorsale de la partie de pied (18) au-delà de la position zéro si, et seulement si, un couple de rotation de cheville ( $\vec{M}$ ) appliqué à la partie de pied (18) est supérieur à une valeur seuil de libération prédéfinie (M$_{\text{libération}}$).

3. Pied prothétique selon l'une des revendications précédentes, **caractérisé en ce que** le dispositif de libération (16) est conçu de manière à ce que la position zéro soit réglable.

4. Pied prothétique selon l'une des revendications précédentes, **caractérisé en ce que** le dispositif d'inhibition (28) comprend

- un premier conduit de fluide (34) à travers lequel le fluide hydraulique (32) s'écoule lorsque la partie de pied (18) exécute un mouvement d'extension dorsale au-delà de la position zéro, le premier conduit de fluide (34) ayant une première résistance à l'écoulement, et

- un conduit de pontage (38) à travers lequel le fluide hydraulique (32) s'écoule lorsque la partie de pied (18) exécute un mouvement d'extension dorsale entre la position de flexion plantaire maximale et la position zéro,
- le conduit de pontage (38) ayant une résistance de pontage à l'écoulement qui est inférieure à la première résistance à l'écoulement.

5. Pied prothétique selon la revendication 4, **caractérisé en ce que** le premier conduit de fluide (34) comprend un clapet anti-retour (50) agencé pour

- empêcher un mouvement d'extension dorsale au-delà de la position zéro tant que le couple rotation de cheville (M) n'est pas supérieur à la valeur seuil de libération ($M_{libération}$), et pour
- libérer le mouvement d'extension dorsale au-delà de la position zéro lorsque le couple de rotation de cheville (M) est supérieur à la valeur seuil de libération.

6. Pied prothétique selon la revendication 4 ou 5, **caractérisé en ce que** le dispositif d'inhibition (28) comprend un deuxième conduit de fluide (36) à travers lequel le fluide hydraulique (32) s'écoule lorsque la partie de pied (18) exécute un mouvement de flexion plantaire, le deuxième conduit de fluide (36) ayant une deuxième résistance à l'écoulement qui est inférieure à la première résistance à l'écoulement.

7. Pied prothétique selon la revendication 6, **caractérisé en ce que** le dispositif de libération (16) comprend un deuxième vérin hydraulique (30) relié au dispositif d'inhibition (28) de telle sorte que

- un mouvement d'extension dorsale au-delà de la position zéro provoque l'écoulement du fluide hydraulique (32) depuis le deuxième vérin hydraulique (30) jusque dans le premier vérin hydraulique (24) à travers le premier conduit de fluide (34),
- un mouvement d'extension dorsale entre la position de flexion plantaire maximale et la position zéro provoque l'écoulement du fluide hydraulique (32) depuis le deuxième vérin hydraulique (30) jusque dans le premier vérin hydraulique (24) à travers le conduit de pontage (38) ; et
- un mouvement de flexion plantaire provoque l'écoulement du fluide hydraulique (32) depuis le premier vérin hydraulique (24) jusque dans le deuxième vérin hydraulique (30) à travers le deuxième conduit de fluide (36).

8. Pied prothétique selon la revendication 7, **caractérisé en ce que** le vérin hydraulique (24) est à double effet et est relié au dispositif d'inhibition (28) de telle sorte que

- un mouvement d'extension dorsale au-delà de la position zéro provoque le retour du fluide hydraulique (32) jusque dans le vérin hydraulique (24) à partir d'un orifice de sortie (90) situé à une extrémité de sortie du vérin hydraulique (24), à travers le premier conduit de fluide (34),
- un mouvement d'extension dorsale entre la position de flexion plantaire maximale et la position zéro provoque l'écoulement du fluide hydraulique (32) à travers un orifice de soutirage (40) et le conduit de pontage (38), et
- un mouvement de flexion plantaire provoque l'écoulement du fluide hydraulique (32) à travers le deuxième conduit de fluide (36) ou le conduit de pontage (38).

9. Pied prothétique selon la revendication 8, **caractérisé en ce que** le conduit de pontage (38) est relié au premier vérin hydraulique (34) ou au deuxième vérin hydraulique (30) de manière à ce qu'un emplacement de soutirage d'un orifice de soutirage soit variable le long d'un axe longitudinal (L) du vérin hydraulique (24) pour ajuster la position zéro.

Fig. 1

EP 3 288 503 B1

Fig. 2a

Fig. 2b

Fig. 3

EP 3 288 503 B1

Fig. 4

Fig. 5

Fig. 6

EP 3 288 503 B1

10

88.1

14

$D_{88.1}$

84

86

88.2

24

88.3

$D_{88.3}$

$D_{88.2}, D_{eff}$

DE

20

Fig. 7

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014057086 A1
- EP 1974699 A1
- US 8709091 B2
- WO 2014039885 A1
- US 20140074255 A1
- US 20140330393 A1